# EUROPEAN PATENT APPLICATION

(11) **EP 3 632 363 A1**
(43) Date of publication of application: **08.04.2020**
(21) Application number: 18198708.2
(22) Date of filing: 04.10.2018
(51) Int. Cl.: A61B 34/20, A61B 8/12, A61B 10/02, A61M 25/00, A61B 17/34, A61B 6/12, A61B 5/00, A61B 10/04, A61B 18/00, A61B 1/267, A61B 6/00, A61B 34/10

(54) **APPARATUS FOR DESIGNING OR CONFIGURING A TRANS-BRONCHIAL NEEDLE GUIDE**

(71) Applicant: Koninklijke Philips N.V., 5656 AG Eindhoven (NL)
(72) Inventor: HAASE, Christian, 5656 AE Eindhoven (NL); GRASS, Michael, 5656 AE Eindhoven (NL)
(74) Representative: de Haan, Poul Erik

(57) **Abstract**

The present invention relates to an apparatus (10) for designing or configuring a guide for a bronchoscope or guiding sheath. It is described to providing (310) a processing unit with at least one diagnostic image of a patient, wherein the at least one diagnostic image comprises image data of a region of interest and image data of the bronchial tree or vascular system in the vicinity of the region of interest. The processing unit determines (320) a position of the region of interest. The processing unit determines (330) a position in the bronchial tree or vascular system from which an intervention is to be carried out via a bronchoscope or guiding sheath. The intervention will comprise movement of a part of an intervention device towards the region of interest, wherein the bronchoscope or guiding sheath is configured such that the part of the intervention device can exit an end of the bronchoscope or guiding sheath. The processing unit determines (340) an angle of movement of the part of the intervention device with respect to a longitudinal axis of the bronchoscope or guiding sheath to guide the part of the intervention device towards the region of interest. The determination comprises utilization of the position of the region of interest and the position in the bronchial tree or vascular system from which the intervention is to be carried out. The processing unit designs (350) or configures (360) a guide to operate in conjunction with the bronchoscope or guiding sheath, the design or configuration comprising utilization of the determined angle of movement of the part of the intervention device. The guide is configured to operate in conjunction with the bronchoscope or guiding sheath to guide the part of the intervention device exiting the end of the bronchoscope or guiding sheath towards the region of interest at the determined angle of movement.

## Description

### FIELD OF THE INVENTION

The present invention relates to an apparatus for designing or configuring a guide for a bronchoscope or guiding sheath, to a guide for a bronchoscope or guiding sheath, to a system for trans-bronchial or vascular intervention, to a method of designing or configuring a guide for a bronchoscope or guiding sheath, to a method for trans-bronchial or vascular intervention, as well as to a computer program element and a computer readable medium.

### BACKGROUND OF THE INVENTION

For the diagnosis and treatment of lung lesions, trans-bronchial biopsies and radio frequency ablation procedures are important tools. The guidance in the bronchial tree is thereby supported by the bronchoscope, CT roadmaps, EM-tracking, or endobronchial ultrasound. During the procedure a bronchoscope or a steerable guiding sheath is placed in front of the lesion that is to be targeted by the biopsy or ablation needle. The needle is then advanced through the bronchoscope or the guiding sheath into the lesion. Due to the limited space inside the bronchi, the bronchoscope as well as the sheath point longitudinally along the bronchi. The lesion can therefore only be entered by the needle at a shallow angle. Lesions at radial locations from the bronchi are accordingly hard to reach. Additionally, it can be difficult to place a Trans-jugular Intrahepatic Portosystemic Shunt (TIPS) at the correct position in a vascular intervention. There is a need to address these issues.

### SUMMARY OF THE INVENTION

It would be advantageous to have improved means of facilitating trans-bronchial interventions and vascular interventions.

The object of the present invention is solved with the subject matter of the independent claims, wherein further embodiments are incorporated in the dependent claims. It should be noted that the following described aspects and examples of the invention apply also to the apparatus for designing or configuring a guide for a bronchoscope or guiding sheath, the guide for a bronchoscope or guiding sheath, the system for trans-bronchial or vascular intervention, the method of designing or configuring a guide for a bronchoscope or guiding sheath, the method for trans-bronchial or vascular intervention with a bronchoscope or guiding sheath, as well as for the computer program element and computer readable medium.

In a first aspect, there is provided an apparatus for designing or configuring a guide for a bronchoscope or guiding sheath, the apparatus comprising:
- an input unit; and
- a processing unit.

The input unit is configured to provide the processing unit with at least one diagnostic image of a patient. The at least one diagnostic image comprises image data of a region of interest and image data of the bronchial tree or vascular system in the vicinity of the region of interest. The processing unit is configured to determine a position of the region of interest and determine a position in the bronchial tree or vascular system from which an intervention is to be carried out via a bronchoscope or guiding sheath. The intervention will comprise movement of a part of an intervention device towards the region of interest. The bronchoscope or guiding sheath is configured such that the part of the intervention device can exit an end of the bronchoscope or guiding sheath. The processing unit is configured to determine an angle of movement of the part of the intervention device with respect to a longitudinal axis of the bronchoscope or guiding sheath to guide the part of the intervention device towards the region of interest, the determination comprising utilization of the position of the region of interest and the position in the bronchial tree or vascular system from which the intervention is to be carried out. The processing unit is configured to design or configure a guide for the bronchoscope or guiding sheath to operate in conjunction with the bronchoscope or guiding sheath. The design or configuration comprises utilization of the determined angle of movement of the part of the intervention device. The guide is configured to operate in conjunction with the bronchoscope or guiding sheath to guide the part of the intervention device exiting the end of the bronchoscope or guiding sheath toward the region of interest at the determined angle of movement.

In this way, the intervention device, such as a needle or TIPS intervention device, can exit a bronchoscope or guiding sheath at a desired angle, that can be significantly deviated from the longitudinal axis of the bronchoscope or guiding sheath, and thus provide an improved access to targeted lesions in trans-bronchial or TIPS interventions.

The guide is designed or configured in a patient specific manner, through utilization of diagnostic imagery to determine the required angle of intervention from the bronchoscope of guiding sheath.

In an example, the guide comprises a part at an angle to the longitudinal axis of the bronchoscope or guiding sheath that is at the angle of movement.

In an example, the guide comprises a channel through which the part of the intervention device can move.

In an example, the configuration of the guide comprises a determined deployment of a balloon or a pull-wire at the end of the bronchoscope or guiding sheath.

In this manner, a standard guide can be used with a fixed exit angle, which can be modified using a balloon at the tip of a wire or a pull wire to change the exit angle manually.

In a second aspect, there is provided a guide for a bronchoscope or guiding sheath. The guide is configured to operate in conjunction with a bronchoscope or guiding sheath to guide a part of an intervention device extending out of an end of the bronchoscope or guiding sheath at a determined angle of movement with respect to a longitudinal axis of the bronchoscope or guiding sheath.

In this way, an intervention device such as a needle used for trans-bronchial intervention or an intervention device used for example for TIPS can exit a bronchoscope or guiding sheath at a desired angle, that can be significantly deviated from the longitudinal axis of the bronchoscope or guiding sheath, and thus provide an improved access to targeted lesions in trans-bronchial interventions or place a Trans-jugular Intrahepatic Portosystemic Shunt (TIPS) at the correct position.

An angle of movement with respect to the longitudinal axis means that there is an angle relative to the longitudinal axis and an angle in the plane perpendicular to the longitudinal axis.

In an example, the guide comprises a part at an angle to the longitudinal axis of the bronchoscope or guiding sheath that is at the angle of movement.

In an example, the guide comprises a channel through which the part of the intervention device can move.

In an example, the guide comprises at least one location marker.

In an example, the guide is configured to guide the part of the intervention device extending at the determined angle of movement with respect to a longitudinal axis of the bronchoscope or guiding sheath through deployment of a balloon or a pull-wire at the end of the bronchoscope or guiding sheath.

In an example, the guide is designed or configured using the apparatus of the first aspect.

In a third aspect, there is provided a system for trans-bronchial or vascular intervention, the system comprising:
- a guide for a bronchoscope or guiding sheath; and
- a bronchoscope or guiding sheath.

The bronchoscope or guiding sheath is configured to carry out an intervention, the intervention comprising movement of a part of an intervention device towards a region of interest, wherein the bronchoscope or guiding sheath is configured such that the part of the intervention device can exit an end of the bronchoscope or guiding sheath. The guide is configured to operate in conjunction with the bronchoscope or guiding sheath to guide the part of the intervention device exiting the end of the bronchoscope or guiding sheath towards the region of interest at a determined angle of movement.

In an example, the guide for a bronchoscope or guiding sheath is a guide designed using the apparatus of the first aspect.

In a fourth aspect, there is provided a method of designing or configuring a guide for a bronchoscope or guiding sheath, the method comprising:
a) providing a processing unit with at least one diagnostic image of a patient, wherein the at least one diagnostic image comprises image data of a region of interest and image data of the bronchial tree or vascular system in the vicinity of the region of interest;
b) determining by the processing unit a position of the region of interest;
c) determining by the processing unit a position in the bronchial tree or vascular system from which an intervention is to be carried out via a bronchoscope or guiding sheath, wherein the intervention will comprise movement of a part of an intervention device towards the region of interest, wherein the bronchoscope or guiding sheath is configured such that the part of the intervention device can exit an end of the bronchoscope or guiding sheath;
d) determining by the processing unit an angle of movement of the part of the intervention device with respect to a longitudinal axis of the bronchoscope or guiding sheath to guide the part of the intervention device towards the region of interest, the determination comprising utilizing the position of the region of interest and the position in the bronchial tree or vascular system from which the intervention is to be carried out; and
e) designing or configuring by the processing unit a guide to operate in conjunction with the bronchoscope or guiding sheath, the design or configuration comprising utilizing the determined angle of movement of the part of the intervention device; wherein the guide is configured to operate in conjunction with the bronchoscope or guiding sheath to guide the part of the intervention device exiting the end of the bronchoscope or guiding sheath towards the region of interest at the determined angle of movement.

In a fifth aspect, there is provided a method for trans-bronchial or vascular intervention with a bronchoscope or guiding sheath. The bronchoscope or guiding sheath comprises a guide. The bronchoscope or guiding sheath is configured to carry out an intervention, the intervention comprising movement of a part of an intervention device towards a region of interest. The bronchoscope or guiding sheath is configured such that the part of the intervention device can exit an end of the bronchoscope or guiding sheath. The method comprises: operating the guide in conjunction with the bronchoscope or guiding sheath and guiding and moving the part of the intervention device exiting the end of the bronchoscope or guiding sheath towards the region of interest at a determined angle of movement.

According to another aspect, there is provided a computer program element controlling apparatus of the first aspect which, if the computer program element is executed by a processing unit, is adapted to perform the method steps of the fourth aspect.

According to another aspect, there is provided a computer readable medium having stored computer element as previously described.

The computer program element, can for example be a software program but can also be a FPGA, a PLD or any other appropriate digital means.

Advantageously, the benefits provided by any of the above aspects equally apply to all of the other aspects and vice versa.

The above aspects and examples will become apparent from and be elucidated with reference to the embodiments described hereinafter.

### BRIEF DESCRIPTION OF THE DRAWINGS

Exemplary embodiments will be described in the following with reference to the following drawings:
Fig. 1 shows a schematic set up of an example of an apparatus for designing or configuring a guide for a bronchoscope or guiding sheath along with an example of a guide designed or configured using the apparatus;
Fig. 2 shows a schematic set up of an example of a system for trans-bronchial or vascular intervention;
Fig. 3 shows a shows a method for designing or configuring a guide for a bronchoscope or guiding sheath; and
Fig. 4 shows on the left needle placement using a normal bronchoscope or guiding sheath and on the right shows needle placement using an example of a trans-bronchial needle guide operating in conjunction with a normal bronchoscope or guiding sheath.

### DETAILED DESCRIPTION OF EMBODIMENTS

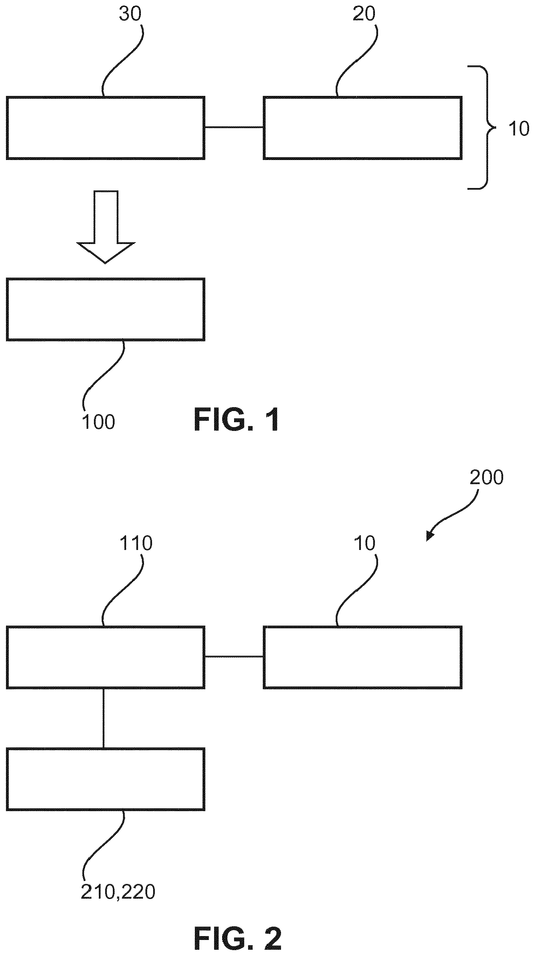

Fig. 1 shows an example of an apparatus 10 for designing or configuring a guide for a bronchoscope or guiding sheath. The apparatus comprises an input unit 20, and a processing unit 30. The input unit is configured to provide the processing unit with at least one diagnostic image of a patient. The at least one diagnostic image comprises image data of a region of interest and image data of the bronchial tree or vascular system in the vicinity of the region of interest. The processing unit is configured to determine a position of the region of interest and determine a position in the bronchial tree or vascular system from which an intervention is to be carried out via a bronchoscope or guiding sheath. The intervention will comprise movement of a part of an intervention device towards the region of interest. The bronchoscope or guiding sheath is configured such that the part of the intervention device can exit an end of the bronchoscope or guiding sheath. The processing unit is configured also to determine an angle of movement of the part of the intervention device with respect to a longitudinal axis of the bronchoscope or guiding sheath to guide the part of the intervention device towards the region of interest. The determination of the angle of movement comprises utilization of the position of the region of interest and the position in the bronchial tree or vascular system from which the intervention is to be carried out. The processing unit is configured also to design or configure a guide for the bronchoscope or guiding sheath to operate in conjunction with the bronchoscope or guiding sheath. The processing is configured to utilize the determined angle of movement of the part of the intervention device to determine the design or configuration. The guide is configured to operate in conjunction with the bronchoscope or guiding sheath to guide the part of the intervention device exiting the end of the bronchoscope or guiding sheath toward the region of interest at the determined angle of movement.

In an example, the intervention device is a needle.

In an example, the treatment comprises insertion of a part of the needle into the lesion.

In an example, the intervention device is a device to be used in a TIPS intervention.

In an example, the region of interest is a lesion to be treated through insertion of a part of a needle into the lesion.

In an example, the region of interest is for example an area between the portal vein and hepatic vein and the intervention comprises advancing a Colapinto needle to connect the portal vein and hepatic vein to create a shunt there between as part of a TIPS procedure.

In an example, the guide is designed as a structure to be affixed to the end of the bronchoscope or guiding sheath.

In this way, a patient specific guide is provided that can be attached to the tip of a bronchoscope or guiding sheath, and provides a predefined exit angle for the intervention device, such as a needle or TIPS intervention device.

In this manner, the shape of the guide can be calculated automatically from the position and direction of the bronchi from which the intervention will exit and the position of the nodule / tumor / lesion under consideration of the structure along the path from the bronchi (vein/artery) to the target.

According to an example, the guide comprises a part at an angle to the longitudinal axis of the bronchoscope or guiding sheath that is at the angle of movement.

According to an example, the guide comprises a channel through which the part of the intervention device can move.

In an example, the structure has a plurality of channels for multiple devices, such as multiple ablation needles, TIPS intervention device and an outlet for a device that facilitates stabilization of the bronchi for example.

In an example, the structure to be affixed to the end of the bronchoscope or guiding sheath is configured such that when affixed to the bronchoscope or guiding sheath can enter the structure and exit the structure at the angle of movement.

In an example, the structure to be affixed to the end of the bronchoscope or guiding sheath comprises a wedge shape, the wedge shape comprising a surface angled at the angle of movement with respect to the longitudinal axis of the bronchoscope or guiding sheath.

In an example, the structure to be affixed to the end of the bronchoscope or guiding sheath is 3D printable.

In an example, the structure to be affixed to the end of the bronchoscope or guiding sheath comprises at least one location marker.

In an example, the at least one location marker comprises at least one X-ray visible marker.

In an example, the at least one location marker is positioned on and/or within the structure such that an orientation of the structure about the longitudinal axis of bronchoscope or guiding sheath can be determined.

In an example, the at least one location marker guide is configured such that the orientation of the guide can be determined from an X-ray image of the guide.

In an example, the at least one location marker is configured such that the orientation of the guide can be determined using a localization system, such as an EM-localization system.

According to an example, the configuration of the guide comprises a determined deployment of a balloon or a pull-wire at the end of the bronchoscope or guiding sheath.

In an example, the determined deployment the balloon or the pull-wire is designed to modify movement of the part of the needle to the angle of movement.

Fig. 1 also shows an example of a guide 100 for a bronchoscope or guiding sheath. The guide can be designed or configured using the apparatus 10 as described above, however this is not essential. The guide is configured to operate in conjunction with a bronchoscope or guiding sheath to guide a part of an intervention device extending out of an end of the bronchoscope or guiding sheath at a determined angle of movement with respect to a longitudinal axis of the bronchoscope or guiding sheath.

In an example, the guide is a trans-bronchial needle guide.

In an example, the guide is configured to be affixed to the end of the bronchoscope or guiding sheath.

According to an example, the guide comprises a part at an angle to the longitudinal axis of the bronchoscope or guiding sheath that is at the angle of movement.

According to an example, the guide comprises a channel through which the part of the intervention device can move.

In an example, the guide is configured such that when affixed to the bronchoscope or guiding sheath the part of the intervention device can enter the guide and exit the guide at the angle of movement.

In an example, the guide comprises a wedge shape, the wedge shape comprising a surface angled at the angle of movement with respect to the longitudinal axis of the bronchoscope or guiding sheath.

In an example, the guide is 3D printable.

According to an example, the guide comprises at least one location marker.

In an example, the at least one location marker comprises at least one X-ray visible marker.

In an example, the at least one location marker is positioned on and/or within the guide such that an orientation of the guide about the longitudinal axis of bronchoscope or guiding sheath can be determined.

In an example, the at least one location marker guide is configured such that the orientation of the guide can be determined from an X-ray image of the guide.

In an example, the at least one location marker is configured such that the orientation of the guide can be determined using a localization system, such as an EM-localization system.

According to an example, the guide is configured to guide the part of the intervention device extending at the determined angle of movement with respect to a longitudinal axis of the bronchoscope or guiding sheath through deployment of a balloon or a pull-wire at the end of the bronchoscope or guiding sheath.

In an example, the guide is configured to deployment the balloon or the pull-wire is to modify movement of the part of the intervention device to the angle of movement.

According to an example, as discussed above the guide is designed or configured using the apparatus 10 as described above.

Fig. 2 shows a system 200 for trans-bronchial or vascular intervention. The system 200 comprises a guide 110 for a bronchoscope or guiding sheath. This can be guide 100 as described above with reference to Fig. 1, and guide 110 need not be designed using the apparatus 10. The system also comprises a bronchoscope 210) or guiding sheath (220. The bronchoscope or guiding sheath is configured to carry out an intervention, the intervention comprising movement of a part of an intervention device towards a region of interest. The bronchoscope or guiding sheath is configured such that the part of the intervention device can exit an end of the bronchoscope or guiding sheath. The guide is configured to operate in conjunction with the bronchoscope or guiding sheath to guide the part of the intervention device exiting the end of the bronchoscope or guiding sheath towards the region of interest at a determined angle of movement.

In an example, the guide is a trans-bronchial needle guide.

In an example, the guide is a guide used in a TIPS intervention.

In an example, the guide, such as a trans-bronchial needle guide or TIPS guide, is affixed to the end of the bronchoscope or guiding sheath.

In an example, the guide is 3D printed directly onto the bronchoscope or guiding sheath, thereby providing a particularly stable connection.

In an example, the guide comprises at least one location marker positioned on and/or within the structure. Thus, since there is a rotational degree of freedom inside the bronchi or vascular system the location markers / structures on / inside the guide can help to detect if the 3D position of the guide is correct to reach the nodule / tumor / lesion. An appropriate location system, such as fluoroscopic X-ray or other EM location system for example can be used to determine the orientation, and the location markers can therefore be markers that are visible in the X-ray region.

In an example, the bronchoscope or guiding sheath comprises a sensor configured to indicate a length of the intervention device, such as needle, that has passed through the guide. In this way, it can be determined when the intervention device has reached its target (the required part of the lesion).

According to an example, the guide for a bronchoscope or guiding sheath is a guide designed using the apparatus 10 as described with respect to Fig. 1.

In an example, the system comprises an image acquisition unit 230 configured to acquire the at least one image.

In an example, the image acquisition unit is a CT X-ray unit for 3D image data. In an example, the image acquisition unit is a cone Beam CT X-ray unit. In an example, the image acquisition unit is a MRI unit.

There is also provided an associated method for trans-bronchial or vascular intervention with a bronchoscope or guiding sheath, wherein the bronchoscope or guiding sheath comprises a guide. The bronchoscope or guiding sheath is configured to carry out an intervention, the intervention comprising movement of a part of an intervention device towards a region of interest. The bronchoscope or guiding sheath is configured such that the part of the intervention device can exit an end of the bronchoscope or guiding sheath. The method comprises operating the guide in conjunction with the bronchoscope or guiding sheath and guiding and moving the part of the intervention device exiting the end of the bronchoscope or guiding sheath towards the region of interest at a determined angle of movement.

In an example, the guide is a trans-bronchial needle guide.

In an example, the guide is a guide used in a TIPS intervention.

In an example, the guide, is as a trans-bronchial needle guide or TIPS guide, and the method comprises affixing the guide to the end of the bronchoscope or guiding sheath.

In an example, the method comprises 3D printing the guide directly onto the bronchoscope or guiding sheath, thereby providing a particularly stable connection.

In an example, the guide comprises at least one location marker positioned on and/or within the structure.

In an example, the method comprises sensing with a sensor of the bronchoscope or guiding sheath a length of the intervention device, such as needle, that has passed through the guide.

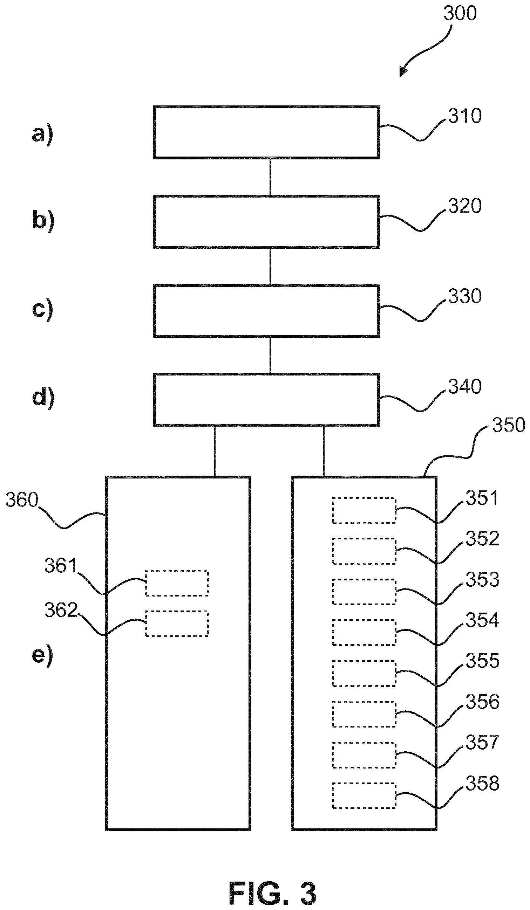

In an example, the guide for a bronchoscope or guiding sheath is a guide designed or configured using the method of designing or configuring a guide for a bronchoscope or guiding sheath as described in conjunction with in Fig. 3, as discussed below.

In an example, the method comprises acquiring with an image acquisition unit configured the at least one image.

In an example, the image acquisition unit is a CT X-ray unit for 3D image data. In an example, the image acquisition unit is a cone Beam CT X-ray unit. In an example, the image acquisition unit is a MRI unit.

Fig. 3 shows a method 300 of designing or configuring a guide for a bronchoscope or guiding sheath in its basic steps. The method comprises:
in a providing step 310, also referred to as step a), providing a processing unit with at least one diagnostic image of a patient, wherein the at least one diagnostic image comprises image data of a region of interest and image data of the bronchial tree or vascular system in the vicinity of the region of interest;
in a determining step 320, also referred to as step b), determining by the processing unit a position of the region of interest;
in a determining step 330, also referred to as step c), determining by the processing unit a position in the bronchial tree or vascular system from which an intervention is to be carried out via a bronchoscope or guiding sheath, wherein the intervention will comprise movement of a part of an intervention device towards the region of interest, wherein the bronchoscope or guiding sheath is configured such that the part of the intervention device can exit an end of the bronchoscope or guiding sheath;
in a determining step 340, also referred to as step d), determining by the processing unit an angle of movement of the part of the intervention device with respect to a longitudinal axis of the bronchoscope or guiding sheath to guide the part of the intervention device towards the region of interest, the determination comprising utilizing the position of the region of interest and the position in the bronchial tree or vascular system from which the intervention is to be carried out; and
in a designing step 350 or configuring step 360, also referred to as step e), designing or configuring by the processing unit a guide to operate in conjunction with the bronchoscope or guiding sheath, the design or configuration comprising utilizing the determined angle of movement of the part of the intervention device;
wherein the guide is configured to operate in conjunction with the bronchoscope or guiding sheath to guide the part of the intervention device exiting the end of the bronchoscope or guiding sheath towards the region of interest at the determined angle of movement.

In an example, the at least one diagnostic image is provided from an input unit.

In an example, the input unit comprises an image acquisition unit 230.

In an example, step e) comprises designing 351 a structure to be affixed to the end of the bronchoscope or guiding sheath.

In an example, step e) comprises designing 352 the structure to be affixed to the end of the bronchoscope or guiding sheath to comprise a part at an angle to the longitudinal axis of the bronchoscope or guiding sheath that is at the angle of movement.

In an example, step e) comprises designing 353 the structure to be affixed to the end of the bronchoscope or guiding sheath to comprise a channel through which the part of the intervention device can move.

In an example, step e) comprises designing 354 the structure to be affixed to the end of the bronchoscope or guiding sheath to be configured such that when affixed to the bronchoscope or guiding sheath the part of the intervention device can enter the structure and exit the structure at the angle of movement.

In an example, step e) comprises designing 355 the structure to be affixed to the end of the bronchoscope or guiding sheath to comprise a wedge shape.

In an example, step e) comprises designing 356 the structure to be affixed to the end of the bronchoscope or guiding sheath as a 3D printable structure.

In an example, step e) comprising designing 357 the structure to be affixed to the end of the bronchoscope or guiding sheath to comprise at least one location marker. In an example, step e) comprising designing the structure to be affixed to the end of the bronchoscope or guiding sheath to comprise at least one X-ray visible marker.

In an example, step e) comprises designing 358 the at least location marker to be positioned on or within the structure such that an orientation of the structure about the longitudinal axis of bronchoscope or guiding sheath can be determined.

In an example, the at least one location marker guide is configured such that the orientation of the guide can be determined from an X-ray image of the guide.

In an example, the at least one location marker is configured such that the orientation of the guide can be determined using a localization system, such as an EM-localization system.

In an example, step e) comprises configuring 361 the guide to provide a determined deployment of a balloon or a pull-wire at the end of the bronchoscope or guiding sheath.

In an example, step e) comprises configuring 362 the guide to provide the determined deployment of the balloon or the pull-wire through modifying movement of the part of the needle to the angle of movement.

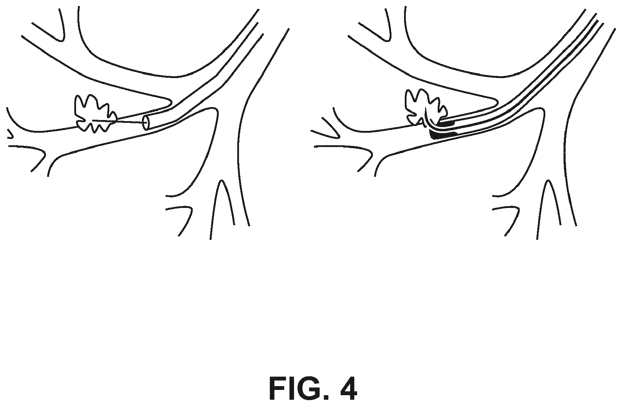

The apparatus for designing or configuring a guide for a bronchoscope or guiding sheath, the guide that can be designed or configured using the apparatus, the system for trans-bronchial or vascular intervention, and associated method, and the method for designing or configuring a guide for a bronchoscope or guiding sheath as described with respect to Figs. 1-3 are now described in more detail with reference to the detailed example described with reference to Fig. 4. In this specific example, the guide is a trans-bronchial needle guide to be used in trans-bronchial interventions. However, the guide has utility in other interventions, such as for the placement of a shunt in a TIPS procedure.

Fig. 4 shows an illustration of a needle placement without (left) and with (right) a trans-bronchial needle guide on the tip of a bronchoscope or guiding sheath. In this example on the right, the trans-bronchial needle guide has been 3D-printed. The illustration shows a bronchial tree with a lesion. In the illustration on the left, the needle (exiting the end of the bronchoscope or guiding sheath) can only partially penetrate the lesion. In the illustration on the right the 3D printed needle guide on the tip of the guiding sheath, allows an improved placement of the needle in the center of the lesion.

The trans-bronchial needle guide shown in Fig. 4 was designed in the following manner:
diagnostic images were recorded, from which the location of the lesion and the bronchi in close proximity were identified. This can be e.g. a CT scan or MRI scan.
procedure path planning was performed to identify a suitable location from which the lesion can be accessed. Information that influences this location is the relative position of the lesion to the bronchi, the shape of the lesion, the desired angle of entrance into the lesion, and the location of blood vessels in the surrounding tissue.

Based on the planned procedure path the required shape for a trans-bronchial needle guide tip is determined. I.e. the orientation and the location of the bronchoscope or the guiding sheath is defined in the planned procedure path. The relative position of the lesion to this orientation and position defines the required exit angle needed from the needle guide tip. Also, the planned type of bronchoscope or guiding sheath influences this step.

The needle guide tip is 3D printed on site, and attached to the bronchoscope or the guiding sheath.

Additionally, the 3D printed guide tip can have defined outlets for multiple devices. E.g. multiple ablation needles, or an outlet for a device that facilitates the stabilization in the bronchi and another outlet for the needle. The guiding tip can also be 3D printed directly onto the guiding sheath to provide a stable connection. Also, the 3D printed guiding tip can contain X-ray opaque markers that allow an accurate visual estimation of its position and its orientation from X-ray angiography images, and live tracking of the guide's position can be carried out if necessary. A precomputed X-ray shadow image of the 3D printed tip can further be used to indicate if the correct orientation and position is reached. An automatic X-ray fluoroscopy based 3D tracking of the tip can also be achieved in this manner. The X-ray opaque markers can be a part of the guiding sheath that connects to the printed model, can be applied as part of the printing process, or they can be part of a matrix onto which the tip is printed.

In another exemplary embodiment, a computer program or computer program element is provided that is characterized by being configured to execute the method steps of the method according to one of the preceding embodiments, on an appropriate system.

The computer program element might therefore be stored on a computer unit, which might also be part of an embodiment. This computing unit may be configured to perform or induce performing of the steps of the method described above. Moreover, it may be configured to operate the components of the above described apparatus and/or system. The computing unit can be configured to operate automatically and/or to execute the orders of a user. A computer program may be loaded into a working memory of a data processor. The data processor may thus be equipped to carry out the method according to one of the preceding embodiments.

This exemplary embodiment of the invention covers both, a computer program that right from the beginning uses the invention and computer program that by means of an update turns an existing program into a program that uses invention.

Further on, the computer program element might be able to provide all necessary steps to fulfill the procedure of an exemplary embodiment of the method as described above.

According to a further exemplary embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. An apparatus (10) for designing or configuring a guide for a bronchoscope or guiding sheath, comprising:
- an input unit (20); and
- a processing unit (30);
wherein, the input unit is configured to provide the processing unit with at least one diagnostic image of a patient, wherein the at least one diagnostic image comprises image data of a region of interest and image data of the bronchial tree or vascular system in the vicinity of the region of interest;
wherein, the processing unit is configured to determine a position of the region of interest and determine a position in the bronchial tree or vascular system from which an intervention is to be carried out via a bronchoscope or guiding sheath, wherein the intervention will comprise movement of a part of an intervention device towards the region of interest, wherein the bronchoscope or guiding sheath is configured such that the part of the intervention device can exit an end of the bronchoscope or guiding sheath;
wherein, the processing unit is configured to determine an angle of movement of the part of the intervention device with respect to a longitudinal axis of the bronchoscope or guiding sheath to guide the part of the intervention device towards the region of interest, the determination comprising utilization of the position of the region of interest and the position in the bronchial tree or vascular system from which the intervention is to be carried out;
wherein, the processing unit is configured to design or configure a guide for the bronchoscope or guiding sheath to operate in conjunction with the bronchoscope or guiding sheath, the design or configuration comprising utilization of the determined angle of movement of the part of the intervention device; and
wherein, the guide is configured to operate in conjunction with the bronchoscope or guiding sheath to guide the part of the intervention device exiting the end of the bronchoscope or guiding sheath toward the region of interest at the determined angle of movement.

2. Apparatus according to claim 1, wherein the guide comprises a part at an angle to the longitudinal axis of the bronchoscope or guiding sheath that is at the angle of movement.

3. Apparatus according to claim 2, wherein the guide comprises a channel through which the part of the intervention device can move.

4. Apparatus according to claim 1, wherein the configuration of the guide comprises a determined deployment of a balloon or a pull-wire at the end of the bronchoscope or guiding sheath.

5. A guide (100, 110) for a bronchoscope or guiding sheath:
wherein, the guide is configured to operate in conjunction with a bronchoscope or guiding sheath to guide a part of an intervention device extending out of an end of the bronchoscope or guiding sheath at a determined angle of movement with respect to a longitudinal axis of the bronchoscope or guiding sheath.

6. Guide according to claim 5, wherein the guide comprises a part at an angle to the longitudinal axis of the bronchoscope or guiding sheath that is at the angle of movement.

7. Guide according to claim 6, wherein the guide comprises a channel through which the part of the intervention device can move.

8. Guide according to any of claims 6-7, wherein the guide comprises at least one location marker.

9. Guide according to claim 5, wherein the guide is configured to guide the part of the intervention device extending at the determined angle of movement with respect to a longitudinal axis of the bronchoscope or guiding sheath through deployment of a balloon or a pull-wire at the end of the bronchoscope or guiding sheath.

10. Guide for a bronchoscope or guiding sheath, wherein the guide (100) is designed or configured using the apparatus of any of claims 1-4.

11. A system (200) for trans-bronchial or vascular intervention, comprising:
- a guide (100, 110) for a bronchoscope or guiding sheath;
- a bronchoscope (210) or guiding sheath (220);
wherein, the bronchoscope or guiding sheath is configured to carry out an intervention, the intervention comprising movement of a part of an intervention device towards a region of interest, wherein the bronchoscope or guiding sheath is configured such that the part of the intervention device can exit an end of the bronchoscope or guiding sheath; and
wherein, the guide is configured to operate in conjunction with the bronchoscope or guiding sheath to guide the part of the intervention device exiting the end of the bronchoscope or guiding sheath towards the region of interest at a determined angle of movement.

12. System according to claim 11, wherein the guide for a bronchoscope or guiding sheath is a guide (100) designed using the apparatus (10) of any of claims 1-4.

13. A method (300) of designing or configuring a guide for a bronchoscope or guiding sheath, comprising:
a) providing (310) a processing unit with at least one diagnostic image of a patient, wherein the at least one diagnostic image comprises image data of a region of interest and image data of the bronchial tree or vascular system in the vicinity of the region of interest;
b) determining (320) by the processing unit a position of the region of interest;
c) determining (330) by the processing unit a position in the bronchial tree or vascular system from which an intervention is to be carried out via a bronchoscope or guiding sheath, wherein the intervention will comprise movement of a part of an intervention device towards the region of interest, wherein the bronchoscope or guiding sheath is configured such that the part of the intervention device can exit an end of the bronchoscope or guiding sheath;
d) determining (340) by the processing unit an angle of movement of the part of the intervention device with respect to a longitudinal axis of the bronchoscope or guiding sheath to guide the part of the intervention device towards the region of interest, the determination comprising utilizing the position of the region of interest and the position in the bronchial tree or vascular system from which the intervention is to be carried out; and
e) designing (350) or configuring (360) by the processing unit a guide to operate in conjunction with the bronchoscope or guiding sheath, the design or configuration comprising utilizing the determined angle of movement of the part of the intervention device;
wherein the guide is configured to operate in conjunction with the bronchoscope or guiding sheath to guide the part of the intervention device exiting the end of the bronchoscope or guiding sheath towards the region of interest at the determined angle of movement.

14. A method for trans-bronchial or vascular intervention with a bronchoscope or guiding sheath, wherein the bronchoscope or guiding sheath comprises a guide, wherein, the bronchoscope or guiding sheath is configured to carry out an intervention, the intervention comprising movement of a part of an intervention device towards a region of interest, and wherein the bronchoscope or guiding sheath is configured such that the part of the intervention device can exit an end of the bronchoscope or guiding sheath; and wherein the method comprises: operating the guide in conjunction with the bronchoscope or guiding sheath and guiding and moving the part of the intervention device exiting the end of the bronchoscope or guiding sheath towards the region of interest at a determined angle of movement.

15. A computer program element for controlling an apparatus according to any one of claims 1 to 4, which when executed by a processor is configured to carry out the method of claim 13.
